Europäisches Patentamt

European Patent Office

Office européen des brevets

(11) Numéro de publication: **0 277 340 B1**

# FASCICULE DE BREVET EUROPEEN

(45) Date de publication de fascicule du brevet: **30.06.93** (51) Int. Cl.⁵: **C12P 7/06**, C12P 7/14

(21) Numéro de dépôt: **87118981.7**

(22) Date de dépôt: **21.12.87**

(54) **Procédé et appareil pour la production d'alcool.**

(30) Priorité: **16.01.87 CH 149/87**

(43) Date de publication de la demande:
**10.08.88 Bulletin 88/32**

(45) Mention de la délivrance du brevet:
**30.06.93 Bulletin 93/26**

(84) Etats contractants désignés:
**AT BE DE FR IT LU NL SE**

(56) Documents cités:
**EP-A- 0 047 641**
**EP-A- 0 049 994**
**EP-A- 0 092 697**
**DE-A- 3 535 050**
**US-A- 4 419 448**

(73) Titulaire: **SOCIETE DES PRODUITS NESTLE S.A.**
**Case postale 353**
**CH-1800 Vevey(CH)**

(72) Inventeur: **Kalina, Vladimir**
**Chemin du Devin 94**
**CH-1012 Lausanne(CH)**

Rank Xerox (UK) Business Services
(3.10/3.6/3.3.1)

**Description**

La présente invention a pour objet un procédé de production d'alcool par fermentation en continu d'un moût dans des fermenteurs disposés en cascade, dans lequel on injecte du moût frais et de l'oxygène en continu au début de la cascade et l'on recueille du moût fermenté en continu à la fin de la cascade, ainsi qu'un appareil pour la production en continu d'alcool, comprenant plusieurs fermenteurs disposés en cascade.

On connaît divers procédés et appareils pour la production d'alcool par fermentation d'un milieu de culture riche en sucre fermentescible que l'on désigne par le terme de moût dans le présent exposé. Parmi les procédés connus, on peut distinguer trois types principaux discutés brièvement ci-après, à savoir le procédé en discontinu en cuve unique, le procédé en continu en cuves disposées en cascade et le procédé en continu en fermenteur à circulation.

Le procédé en discontinu en cuve unique est encore utilisé aujourd'hui en raison de sa simplicité. Son principal désavantage est sa faible productivité.

Le procédé en continu en cuves disposées en cascade, dans lequel on injecte du moût frais et de l'oxygène en continu au début de la cascade, la levure circule avec le moût d'une cuve à l'autre après avoir été produite ou introduite dans la première cuve et l'on recueille du moût fermenté en continu à la fin de la cascade, ne présente guère une productivité plus grande que celle du procédé discontinu en cuve unique mais il évite les interruptions de production et les nettoyages de cuve entre deux lots de production.

Le procédé en continu en fermenteur à circulation se distingue par sa grande productivité due au fait qu'il permet d'utiliser une grande concentration de levure, floculante en l'occurence, dans le moût. Dans ce type de procédé connu, la concentration de levure dans le moût est maintenue à la valeur désirée par un recyclage d'une partie au moins de la levure séparée du moût fermenté soutiré. Or, un tel recyclage a pour conséquence un long temps de séjour moyen de la levure dans le fermenteur.

La présente invention a pour but de proposer un procédé et un appareil pour la production d'alcool par fermentation en continu qui présentent une grande productivité tout en permettant d'éviter tout recyclage de la levure de manière à assurer un bref temps de séjour moyen de la levure dans le moût durant la fermentation.

A cet effet, le procédé selon la présente invention est caractérisé par le fait que l'on utilise une levure floculante, on favorise la croissance de la levure dans un premier fermenteur à circulation, on inhibe la croissance de la levure dans au moins un fermenteur à circulation suivant, on sépare la levure du moût fermenté à la fin de ladite cascade et on écarte la levure séparée.

De même, l'appareil selon la présente invention est caractérisé par le fait qu'il comprend plusieurs fermenteurs à circulation et un décanteur disposés en ladite cascade.

On a constaté en effet avec surprise qu'il est ainsi possible de réaliser une fermentation avec une grande concentration de levure dans le moût sans qu'il soit nécessaire de recycler la levure séparée du moût fermenté pour maintenir cette concentration à la valeur désirée.

On a constaté en particulier que l'usage de fermenteurs à circulation disposés en cascade permet de réaliser d'un part les conditions de circulation internes du moût dans chaque fermenteur qui permettent l'usage d'une levure floculante et d'autre part les conditions de circulation globale du moût au travers de ladite cascade qui permettent un renouvellement adéquat de la masse de levure dans chaque fermenteur. Ces deux types de circulation dites interne et globale peuvent être ainsi réalisées et contrôlées chacune de manière pratiquement indépendante alors que d'une part elles sont confondues sur la plus grande partie du circuit interne propre à chaque fermenteur et d'autre part elles impliquent des débits de moût très différents, à savoir un débit relativement grand pour la circulation interne et un débit relativement faible pour la circulation globale.

En conséquence de ce qui précède, on a constaté qu'il est ainsi possible de réduire à quelques dizaines d'heures le temps de séjour moyen de la levure dans le moût durant la fermentation tout en assurant une productivité de l'ordre de 20 g d'alcool par h et par l de moût dans l'appareil.

L'importance de la réduction du temps de séjour moyen de la levure dans le moût se mesure au risque de contamination du moût par des microorganismes indésirables. Même si dans certaines conditions de fermentation traditionnelles la plupart des microorganismes voient leur vitesse de croissance fortement ralentie et ne peuvent donc pas contaminer le moût, il existe des microorganismes capables de se multiplier à partir de spores et d'envahir n'importe quelle installation de fermentation en l'espace de 3 semaines à 1 mois par exemple. Si l'on désire travailler en continu durant de longues périodes sans courir de risque de contamination, il est donc essentiel de réduire le temps de séjour moyen de la levure dans le moût de manière draconienne.

Pour mettre en oeuvre le procédé selon la présente invention, on réalise de préférence la fermentation sous une pression de gaz carbonique d'au moins un bar qui exerce un effet favorable sur l'activité physiologique de la levure ainsi que sur ses aptitudes à la floculation.

On peut introduire au départ dans les fermenteurs et y maintenir ensuite une quantité de levure telle que le moût présente de préférence une teneur en levure de 30-70 g en poids sec par l.

On peut produire dans le premier fermenteur la plus grande partie du total de la levure produite durant la fermentation. Pour ce faire, on peut injecter dans le premier fermenteur du moût frais contenant 70-100 g de sucre fermentescible par l, à raison de 0,8-1,0 volume de moût frais par volume de moût contenu dans le fermenteur et par heure, autrement dit à un taux de dilution horaire de 0,8-1,0. De cette façon, on limite la teneur en alcool du moût dans le premier fermenteur à une valeur de 30-40 g/l qui permet encore la croissance désirée de la levure.

On peut favoriser la croissance de la levure dans le premier fermenteur à circulation en y injectant une quantité d'oxygène gale ou peu supérieure à la seule quantité d'oxygène nécessaire à cette croissance, de préférence 0,05-0,15 ml d'oxygène à pression atmosphérique par g de poids sec de levure et par h dans le moût, de manière à permettre une résorbtion totale de l'oxygène dans le moût. L'usage de cette seule quantité minimale d'oxygène est rendu possible en particulier par l'effet d'une variation périodique ou pulsation de la concentration d'oxygène que la levure rencontre dans le moût grâce notamment à la circulation interne du moût dans le fermenteur.

On peut inhiber la croissance de la levure dans lesdits fermenteurs à circulation suivants en n'y injectant aucun oxygène, et/ou en limitant la concentration de phosphate assimilable dans le moût par exemple. Il n'y a en effet pas besoin d'une aération dans les fermenteurs suivants. L'oxygène estant dans le moût et les métabolites intermédiaires produits dans le premier fermenteur sont suffisants pour que la production d'alcool par fermentation se poursuive dans les fermenteurs suivants.

C'est ainsi que l'on peut injecter dans chacun desdits fermenteurs suivants d'une part du moût frais contenant 150-250 g de sucre fermentescible par l à un taux de dilution horaire de 0,04-0,4 et d'autre part du moût fermenté transféré du fermenteur précédent en quantité horaire égale à la quantité horaire totale du moût injecté dans ce fermenteur précédent. Ce faisant, on peut élever progressivement la teneur en alcool du moût dans lesdits fermenteurs suivants et obtenir une teneur en alcool du moût de 55-65 g/l à la fin de ladite cascade.

Enfin, on peut séparer la levure du moût fermenté par décantation à la fin de ladite cascade. De préférence, on sépare la levure du moût fermenté dans un décanteur sous pression de gaz carbonique, la fermentation pouvant s'achever dans ce décanteur. La levure séparée dans le décanteur où elle peut convertir en alcool les restes de sucre fermentescible en provenance d'un dernier fermenteur à circulation n'est donc pas recyclée mais au contraire écartée, de manière à limiter radicalement le temps de séjour moyen de la levure dans le moût. Ce temps de séjour moyen est alors pratiquement égal au quotient de la quantité totale de levure présente dans les fermenteurs et le décanteur divisée par la quantité de levure produite par h durant la fermentation. Le temps moyen mis par le moût frais injecté dans le premier fermenteur pour traverser ladite cascade est généralement nettement plus court que ce temps de séjour moyen de la levure dans le moût, ce qui renforce la stabilité à long terme des présents procédé et appareil, autrement dit leur sécurité en matière de contamination éventuelle par des microorganismes sporulants.

L'appareil selon la présente invention est donc caractérisé par le fait qu'il comprend plusieurs fermenteurs à circulation et un décanteur disposés en cascade. Les fermenteurs à circulation peuvent être de tout type dans lequel d'une part le moût peut circuler en circuit fermé et d'autre part les conditions régnant dans une zone ou cuve de fermentation permettent l'usage d'une levure floculante.

Un type de fermenteur à circulation qui se prête particulièrement bien à la réalisation du présent appareil est celui qui comprend une cuve de fermentation surmontée d'une pompe mammouth. Dans ce type de fermenteur, le moût peut circuler grâce au seul gaz carbonique dégagé durant la fermentation. Le gaz carbonique est maintenu en solution sous pression dans la cuve par une vanne de contre-pression et il exerce l'effet de pompage en se détendant dans la conduite ou pompe mammouth située au-dessus de la vanne. De l'oxygène peut être injecté en quantités minimes, sous forme de mélange éventuel avec de l'azote et/ou du gaz carbonique, dans la partie supérieure d'une conduite de retour reliant le sommet de la pompe mammouth au bas de la cuve de fermentation, de manière à être complètement résorbé dans le moût avant de pénétrer dans la cuve.

C'est ainsi qu'une forme de réalisation préférée du présent appareil est caractérisée par le fait que chaque fermenteur à circulation comprend une cuve de fermentation, une vanne de contre-pression au sommet de la cuve, une pompe mammouth au-dessus de la vanne de contre-pression et une conduite de retour reliant le sommet de la pompe mammouth à la partie inférieure de la cuve, le premier fermenteur présentant un dispositif d'injection de gaz dans la partie supérieure de ladite conduite de retour et chaque fermenteur présentant un dispositif d'injection de moût frais et/ou transféré ainsi qu'un dispositif de transfert de moût fermenté branchés sur la partie inférieure de ladite conduite de retour, et le décanteur comprend une cuve de décantation sous pression reliée par son sommet à une conduite de soutirage de moût fermenté et par son fond à une conduite de vidange de levure décantée, le dispositif de transfert de moût fermenté dudit dernier fermenteur étant relié à un dispositif d'injection de moût frais et/ou transféré branché sur la cuve de décantation.

Le nombre des fermenteurs à circulation peut être de 2 à 6 par exemple. Leur volume peut être important. Il est compris de préférence entre 50 m$^3$ et plusieurs centaines de m$^3$. La cuve présente de préférence une partie centrale cylindrique, une partie inférieure ou fond hémisphérique et une partie supérieure hémisphérique. La section horizontale de ladite partie centrale présente de préférence une dimension telle que la vitesse ascentionnelle du moût dans la cuve soit de environ 0,5-2 cm/s. La hauteur de la pompe mammouth est de préférence telle qu'une surpression d'au moins 1 bar soit encore exercée dans la partie supérieure de la cuve et qu'une pression de environ 0,5 bar soit à disposition pour faire circuler le moût à un débit correspondant à environ 5-10 fois le volume du fermenteur par h en surmontant la résistance de l'ensemble du circuit. Pour une rétention de gaz de environ 35% dans la pompe mammouth par exemple, ceci signifie une hauteur totale de la pompe de au moins 15 m environ.

La résistance du circuit est due en particulier à l'échangeur de chaleur, du type tubulaire de préférence, qu'il faut prévoir, par exemple sur la conduite de retour, pour maintenir une température de environ 30-35°C dans la cuve de fermentation, avec une différence ne dépassant pas environ 3°C entre le haut et le bas de la cuve.

Enfin, la cuve du décanteur peut présenter par exemple un corps cylindrique fermé sur le dessus par un couvercle plat ou légèrement bombé et sur le dessous par un fond conique. Le volume de cette cuve peut être de l'ordre du tiers ou de la moitié du volume des cuves des fermenteurs.

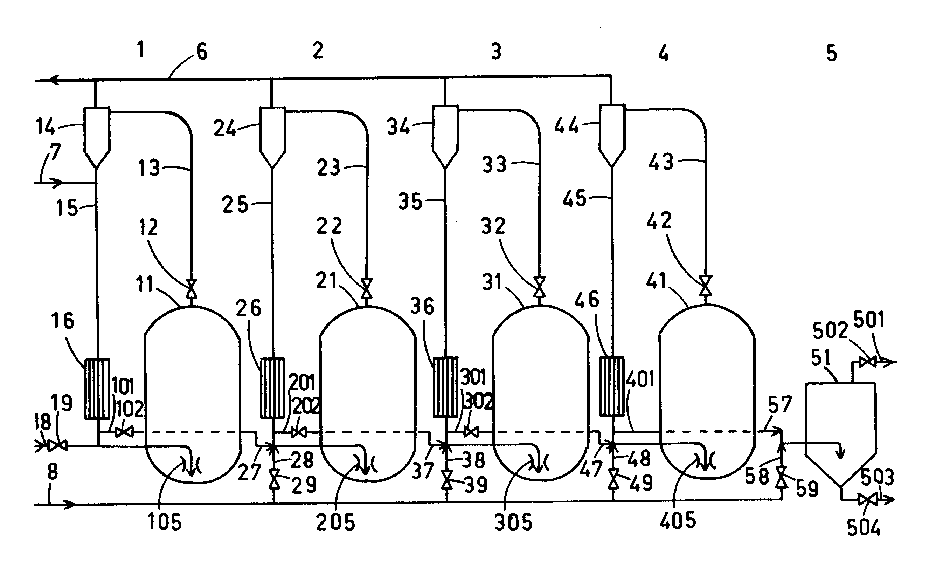

Le présent appareil est décrit ci-après en référence au dessin annexé qui en représente schématiquement une forme d'exécution.

Dans la forme d'exécution représentée au dessin, l'appareil comprend quatre fermenteurs à circulation 1-4 et un décanteur 5 disposés en cascade. Chaque fermenteur à circulation comprend une cuve de fermentation 11-41 présentant une partie centrale cylindrique fermée sur le dessous et le dessus par une partie inférieure et une partie supérieure hémisphériques.

Chaque cuve 11-41 présente à son sommet une vanne de contre-pression 12-42 par laquelle elle communique avec une pompe mammouth 13-43 située au-dessus d'elle. Chaque pompe mammouth est une simple colonne ou conduite de pompage qui débouche à son sommet sur un dispositif de dégazage du type cyclone 14-44.

La partie supérieure de chaque cyclone est reliée à une conduite d'évacuation 6 du gaz carbonique alors que sa partie inférieure est reliée à une conduite de retour 15-45 qui relie ainsi le sommet de la pompe mammouth 13-43 à la partie inférieure de la cuve 11-41.

Le premier fermenteur présente un dispositif d'injection de gaz sous forme d'une conduite d'injection de gaz 7 branchée sur la partie supérieure de la conduite de retour 15. Chaque conduite de retour 15-45 traverse dans sa partie inférieure un échangeur de chaleur tubulaire 16-46.

Chaque fermenteur présente un dispositif d'injection de moût frais et/ou transféré branché sur la partie inférieure de la conduite de retour 15-45, au-dessous de l'échangeur de chaleur 16-46. Pour le premier fermenteur, le dispositif comprend une conduite d'injection de moût frais dilué 18 et une vanne d'injection de moût frais dilué 19. Pour les fermenteurs suivants, ce dispositif comprend une conduite d'injection de moût transféré 27-47 et une conduite d'injection de moût frais concentré 28-48 reliée à une conduite d'approvisionnement en moût frais concentré 8 par une vanne d'injection de moût frais concentré 29-49.

Chaque fermenteur présente en outre un dispositif de transfert de moût fermenté branché sur la conduite de retour au-dessous de l'échangeur de chaleur 16-46. Pour les trois premiers fermenteurs, ce dispositif comprend une conduite de transfert de moût fermenté 101-301 reliée par l'intermédiaire d'une vanne de transfert 102-302 à la conduite d'injection de moût transféré 27-47 du fermenteur suivant. Pour le dernier fermenteur, ce dispositif consiste en une simple conduite de transfert de moût fermenté 401 directement reliée à une conduite d'injection de moût transféré 57 du décanteur 5.

Enfin, chaque conduite de retour 15-45 débouche dans la partie inférieure de la cuve 11-41 au travers d'un dispositif de brassage du type Venturi 105-405.

Le décanteur 5 comprend une cuve 51 de décantation sous pression présentant un corps cylindrique fermé sur le dessus par un couvercle plat et sur le dessous par un fond conique. La cuve 51 est reliée par son sommet ou couvercle à une conduite 501 de soutirage de moût fermenté, par l'intermédiaire d'une vanne de soutirage 502. La cuve 51 est reliée par son fond à une conduite de vidange 503, par l'intermédiaire d'une vanne de vidange 504.

Le décanteur 5 présente également un dispositif d'injection de moût frais et/ou transféré branché sur la partie inférieure du corps cylindrique de la cuve de décantation. Ce dispositif comprend la conduite d'injection 57 de moût transféré du dernier fermenteur et une conduite d'injection 58 de moût frais concentré reliée à la conduite d'approvisionnement 8 par une vanne d'injection 59.

L'exemple ci-après illustre un mode d'exécution du présent procédé de production d'alcool par fermentation en continu.

Exemple

Pour réaliser une fermentation en continu selon le présent procédé, on utilise un appareil semblable à celui décrit ci-dessus en référence au dessin annexé.

Dans cet appareil, les quatre fermenteurs présentent chacun un volume total de 100 m³ et une hauteur totale de 24 m. Le décanteur présente un volume de 50 m³. Les cuves des fermenteurs et du décanteur présentent un diamètre de 5 m.

Après une phase initiale de lancement durant laquelle on introduit et/ou produit la quantité nécessaire de levure floculante Saccharomyces cerevisiae CBS 2961 dans les cuves, on réalise le procédé de fermentation en continu dans les conditions réunies au tableau ci-après:

## Tableau

| Fermenteur (No) | 1 | 2 | 3 | 4 | Décanteur |
|---|---|---|---|---|---|
| Quantité de moût frais injecté (m³/h) | 90 | 28,8 | 23,4 | 16,2 | 4,5 |
| Teneur en sucre fermentescible du moût frais injecté (g/l) | 85 | 181 | 181 | 181 | 181 |
| Taux de dilution par le moût frais injecté (h$^{-1}$) | 0,9 | 0,288 | 0,234 | 0,162 | 0,09 |
| Taux de dilution horaire total (h$^{-1}$) | 0,9 | 1,188 | 1,422 | 1,584 | 3,260 |
| Teneur du moût en poids sec de levure dans les cuves (g/l) | 36,1 | 36,0 | 35,4 | 34,9 | 60 |
| Temps de séjour moyen de la levure dans les cuves (h) | 11,6 | 8,9 | 8,2 | 8,0 | 7,5 |
| Teneur en alcool du moût (g/l) | 36,9 | 47,8 | 53,5 | 57,5 | 60 |
| Quantité d'oxygène à pression atmosphérique injectée sous forme d'air (m³/min) | 0,2 | - | - | - | - |
| Vitesse de circulation interne du moût (m³/h) | 700 | 500 | 500 | 500 | - |

Dans ces conditions, la vitesse de circulation globale du moût, autrement dit le débit total de l'appareil est de 163 m$^3$/h. La production totale d'alcool, y-compris celui que l'on récupère du $CO_2$ évacué par les cyclones est de 9,63 t/h, ce qui représente une productivité de 21,4 g d'alcool par h et par litre de volume total de l'appareil. La quantité totale de levure produite est de 400 kg/h qui sont écartés sous forme de 3,63 m$^3$/h d'une suspension contenant 110 g de poids sec de levure par litre.

La fermentation peut se poursuivre ainsi durant plusieurs mois sans risque de contamination par un microorganisme sporulant.

**Revendications**

1. Procédé de production d'alcool par fermentation en continu d'un moût dans des fermenteurs disposés en cascade, dans lequel on injecte du moût frais et de l'oxygène n continu au début de la cascade et l'on recueille du moût fermenté en continu à la fin de la cascade, caractérisé par le fait que l'on utilise une levure floculante, on favorise la croissance de la levure dans un premier fermenteur à circulation, on inhibe la croissance de la levure dans au moins un fermenteur à circulation suivant, on sépare la levure du moût fermenté à la fin de ladite cascade et on écarte la levure séparée.

2. Procédé selon la revendication 1, caractérisé par le fait que l'on réalise la fermentation sous une pression de gaz carbonique d'au moins 1 bar.

3. Procédé selon la revendication 1, caractérisé par le fait que le moût présente une teneur en levure de 30-70 g en poids sec par l dans les fermenteurs.

4. Procédé selon la revendication 1, caractérisé par le fait que l'on injecte du moût frais contenant 70-100 g de sucre fermentescible par l dans le premier fermenteur, à un taux de dilution horaire de 0,8-1,0.

5. Procédé selon la revendication 1, caractérisé par le fait que l'on injecte par heure dans le premier fermenteur 0,05-0,15 ml d'oxygène pression atmosphérique par g de poids sec de levure dans le moût.

6. Procédé selon la revendication 1, caractérisé par le fait que l'on injecte dans chacun desdits fermenteurs suivants d'une part du moût frais contenant 150-250 g de sucre fermentescible par 1 à un taux de dilution horaire de 0,04-0,4 et d'autre part du moût fermenté transféré du fermenteur précédent en quantité horaire égale à la quantité horaire totale du moût injecté dans ce fermenteur précédent.

7. Procédé selon la revendication 1, caractérisé par le fait que la teneur en alcool du moût est de 30-40 g/l dans le premier fermenteur et de 55-65 g/l à la fin de ladite cascade.

8. Procédé selon la revendication 1, caractérisé par le fait que l'on sépare la levure du moût fermenté dans un décanteur sous pression où l'on achève la fermentation.

9. Appareil pour la production d'alcool en continu, comprenant plusieurs fermenteurs disposés en cascade, caractérisé par le fait qu'il comprend plusieurs fermenteurs à circulation et un décanteur disposés en ladite cascade, chaque fermenteur à circulation comprenant une cuve de fermentation, une vanne de contre-pression au sommet de la cuve, une pompe mammouth au-dessus de la vanne de contre-pression et une conduite de retour reliant le sommet de la pompe mammouth à la partie inférieure de la cuve, le premier fermenteur présentant un dispositif d'injection de gaz dans la partie supérieure de ladite conduite de retour et chaque fermenteur présentant un dispositif d'injection de moût frais et/ou transféré ainsi qu'un dispositif de transfert de moût fermenté branchés sur la partie inférieure de ladite conduite de retour, et le décanteur comprenant une cuve de décantation sous pression reliée par son sommet à une conduite de soutirage de moût fermenté et par son fond à une conduite de vidange de levure décantée, le dispositif de transfert de moût fermenté dudit dernier fermenteur étant relié à un dispositif d'injection de moût frais et/ou transféré branché sur la partie inférieure de la cuve de décantation.

**Claims**

1. A process for the production of alcohol by continuous fermentation of a must in fermenters arranged in a cascade, in which fresh must and oxygen are continuously introduced at the beginning of the

6

cascade and fermented must is continuously collected at the end of the cascade, characterised in that a flocculating yeast is used, the growth of the yeast is promoted in a first circulation fermenter, the growth of the yeast is inhibited in at least one following circulation fermenter, the yeast is separated from the fermented must at the end of the cascade and the yeast separated is removed.

2. A process as claimed in Claim 1, characterised in that the fermentation is carried out under a carbon dioxide pressure of at least 1 bar.

3. A process as claimed in Claim 1 or 2, characterised in that the must has a yeast content of 30 to 70 g dry weight per litre in the fermenters.

4. A process as claimed in any of Claims 1 to 3, characterised in that fresh must containing 70 to 100 g of fermentable sugar per litre is introduced into the first fermenter at an hourly dilution rate of 0.8 to 1.0.

5. A process as claimed in any of Claims 1 to 4, characterised in that from 0.05 to 0.15 ml oxygen at atmospheric pressure per g dry weight of yeast in the must is introduced hourly into the first fermenter.

6. A process as claimed in any of Claims 1 to 5, characterised by the introduction into each of said following fermenters of, on the one hand, fresh must containing 150 to 250 g fermentable sugar per litre at an hourly dilution rate of from 0.04 to 0.4 and, on the other hand, of fermented must transferred from the preceding fermenter in an hourly quantity equal to the total hourly quantity of the must introduced into said preceding fermenter.

7. A process as claimed in any of Claims 1 to 6, characterised in that the alcohol content of the must is 30 to 40 g/l in the first fermenter and 55 to 65 g/l at the end of the cascade.

8. A process as claimed in any of Claims 1 to 7, characterised in that the yeast is separated from the fermented must in a decanter under pressure in which fermentation is completed.

9. A process as claimed in any of Claims 1 to 8, wherein it is carried out in an apparatus comprising several fermenters arranged in a cascade, characterised in that it comprises several circulation fermenters and a decanter arranged in a cascade, each circulation fermenter comprising a fermentation vat, a counter-pressure valve at the top of the vat, an airlift pump above the counter-pressure valve and a return pipe connecting the top of the airlift pump to the lower part of the vat, the first fermenter comprising means for introducing gas into the upper part of said return pipe and each fermenter comprising means for introducing fresh and/or transferred must and means for transferring fermenter must connected to the lower part of said return pipe, while the decanter comprises a decantation vat under pressure connected at its top to a pipe for the removal of fermented must and, at its base, to a pipe for emptying decanted yeast, the means for transferring fermented must of said last fermenter being connected to means for introducing fresh and/or transferred must connected to the lower part of the decantation vat.

**Patentansprüche**

1. Verfahren zur Herstellung von Alkohol durch kontinuierliches Vergären einer Maische in Fermentern, die in einer Kaskade angeordnet sind, bei welchem Verfahren frische Maische und Sauerstoff kontinuierlich am Beginn der Kaskade eingeführt und vergorene Maische kontinuierlich am Ende der Kaskade abgezogen wird, dadurch gekennzeichnet, daß eine Flockhefe verwendet wird, daß das Wachstum der Hefe in einem ersten Umlauffermenter begünstigt wird, daß das Wachstum der Hefe in wenigstens einem nachfolgenden Umlauffermenter gehemmt wird, daB die Hefe von der vergorenen Maische am Ende der Kaskade abgetrennt wird und die abgetrennte Hefe entfernt wird.

2. Verfahren nach Anspruch 1, dadurch gekennzeichnet, daß die Gärung unter einem Kohlendioxiddruck von wenigstens 1 bar durchgeführt wird.

3. Verfahren nach Anspruch 1, dadurch gekennzeichnet, daß die Maische in den Fermentern einen Hefegehalt von 30 bis 70 g/l, bezogen auf die Trockensubstanz, aufweist.

**4.** Verfahren nach Anspruch 1, dadurch gekennzeichnet, daß in den ersten Fermenter eine frische Maische eingeführt wird, welche 70 bis 100 g/l an vergärbarem Zucker enthält, bei einem stündlichen Verdünnungsverhältnis von 0,8 bis 1,0.

**5.** Verfahren nach Anspruch 1, dadurch gekennzeichnet, daß pro Stunde in den ersten Fermenter 0,05 bis 0,15 ml Sauerstoff von Atmosphärendruck pro g Trockensubstanz der Hefe in der Maische eingeführt werden.

**6.** Verfahren nach Anspruch 1, dadurch gekennzeichnet, daß in jeden der nachfolgenden Fermenter einerseits frische Maische mit einem Gehalt von 150 bis 250 g/l an vergärbarem Zucker, bei einem stündlichen Verdünnungsverhältnis von 0,04 bis 0,4, und anderseits vergorene Maische eingeführt wird, welche aus dem vorhergehenden Fermenter in einer stündlichen Menge übergeführt wird, welche gleich groß wie die pro Stunde in diesen vorhergehenden Fermenter eingeführte Gesamtmaischemenge ist.

**7.** Verfahren nach Anspruch 1, dadurch gekennzeichnet, daß der Alkoholgehalt der Maische im ersten Fermenter 30 bis 40 g/l und am Ende der genannten Kaskade 55 bis 65 g/l beträgt.

**8.** Verfahren nach Anspruch 1, dadurch gekennzeichnet, daß die Hefe von der vergorenen Maische in einer Dekantiervorrichtung unter Druck abgetrennt wird, in welcher die Gärung vollendet wird.

**9.** Vorrichtung zur kontinuierlichen Herstellung von Alkohol, die mehrere, in einer Kaskade angeordnete Fermenter umfaßt, dadurch gekennzeichnet, daß sie mehrere in der genannten Kaskade angeordnete Umlauffermenter sowie eine in dieser Kaskade angeordnete Dekantiervorrichtung aufweist, wobei jeder Umlauffermenter ein Gärgefäß, ein Gegendruckventil im Kopf des Gärgefäßes, eine Mammutpumpe oberhalb des Gegendruckventiles und eine Rückleitung umfaßt, welche den Kopf der Mammutpumpe mit dem Unterteil des Gärgefäßes verbindet, wobei der erste Fermenter im oberen Teil der genannten Rückleitung eine Vorrichtung zum Einführen von Gas aufweist, wobei jeder Fermenter eine Vorrichtung zum Einführen von frischer und/oder übergeführter Maische sowie eine Vorrichtung zum Überführen von vergorener Maische aufweist, welche Vorrichtungen mit dem Unterteil der genannten Rückleitung verbunden sind, und wobei die Dekantiervorrichtung ein unter Druck stehendes Dekantiergefäß aufweist, das über seinen Kopf mit einer Leitung zum Abziehen von vergorener Maische und über seinen Boden mit einer Leitung zum Entleeren der dekantierten Hefe verbunden ist, wobei die Vorrichtung zum Überführen der vergorenen Maische aus dem letzten Fermenter mit einer Vorrichtung zum Einführen von frischer und/oder übergeführter Maische verbunden ist, welche an den Unterteil des Dekantiergefäßes angeschlossen ist.